(19) Europäisches Patentamt
European Patent Office
Office européen des brevets

(11) Veröffentlichungsnummer: **0 154 906**
**A2**

(12) # EUROPÄISCHE PATENTANMELDUNG

(21) Anmeldenummer: **85102290.5**

(22) Anmeldetag: **01.03.85**

(51) Int. Cl.⁴: **C 07 D 251/38**, C 07 D 251/46, A 01 N 43/64

(30) Priorität: **13.03.84 DE 3409065**

(43) Veröffentlichungstag der Anmeldung: **18.09.85 Patentblatt 85/38**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Dickoré, Karlfried, Dr., Nicolai-Hartmann-Strasse 19, D-5090 Leverkusen 1 (DE)**
Erfinder: **Steinbeck, Karl, Dr., Rosenkranz 36, D-5093 Burscheid (DE)**
Erfinder: **Eue, Ludwig, Dr., Paul-Klee-Strasse 36, D-5090 Leverkusen 1 (DE)**
Erfinder: **Schmidt, Robert Rudolf, Dr., Im Waldwinkel 110, D-5060 Bergisch Gladbach 2 (DE)**

(54) **3-Cyclopropylmethyl-1,3,5-triazin-2,4-dione.**

(57) Die Erfindung betrifft neue 3-Cyclopropylmethyl-1,3,5-triazin-2,4-dione der allgemeinen Formel

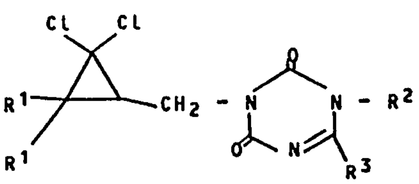

(I)

in welcher
$R^1$ für Wasserstoff oder Alkyl steht,

$R^2$ für Alkyl, für Amino oder für einen Rest $-N=C<^{R^4}_{R^5}$

steht, wobei $R^4$ und $R^5$ unabhängig voneinander für Wasserstoff, für Alkyl oder für gegebenenfalls substituiertes Aryl stehen, oder gemeinsam für einen zweifach verknüpften Alkandiylrest stehen, und

$R^3$ für Alkylthio, Alkylamino oder Dialkylamino steht, verschiedene Verfahren und Zwischenprodukte zu ihrer Herstellung sowie ihre Verwendung als Herbizide.

EP 0 154 906 A2

BAYER AKTIENGESELLSCHAFT 5090 Leverkusen, Bayerwerk
Konzernverwaltung RP
Patentabteilung Bi/ABc
Ib

3-Cyclopropylmethyl-1,3,5-triazin-2,4-dione

Die Erfindung betrifft neue 3-Cyclopropylmethyl-1,3,5-triazin-2,4-dione, mehrere Verfahren zu ihrer Herstellung und ihre Verwendung als Herbizide.

Es ist bereits bekannt, daß bestimmte 1,3,5-Triazin-2,4-dione, wie beispielsweise das 1-Amino-6-methylthio-3-phenyl-1,3,5-triazin-2,4-dion, herbizide Eigenschaften besitzen (vgl. z.B. DE-OS 22 54 200 /Le A 14 692/).

Die herbizide Wirkung dieser Verbindungen gegenüber Schadpflanzen, ebenso wie deren Verträglichkeit gegenüber wichtigen Kulturpflanzen, ist jedoch nicht immer in allen Anwendungsbereichen völlig zufriedenstellend.

Es wurden neue 3-Cyclopropylmethyl-1,3,5-triazin-2,4-dione der allgemeinen Formel (I),

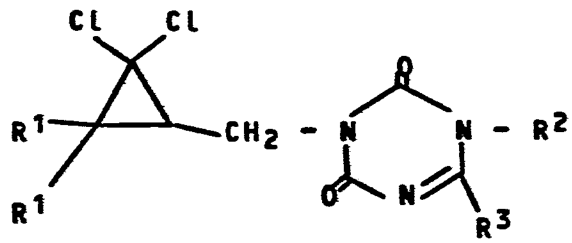

(I)

in welcher

$R^1$ für Wasserstoff oder Alkyl steht,

$R^2$ für Alkyl, für Amino oder für einen Rest

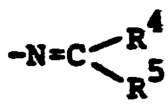 steht, wobei $R^4$ und $R^5$ unabhängig voneinander für Wasserstoff, für Alkyl oder für gegebenenfalls substituiertes Aryl stehen, oder gemeinsam für einen zweifach verknüpften Alkandiylrest stehen, und

$R^3$ für Alkylthio, Alkylamino oder Dialkylamino steht,

gefunden.

Weiterhin wurde gefunden, daß man die neuen 3-Cyclopropylmethyl-1,3,5-triazin-2,4-dione der allgemeinen Formel (I),

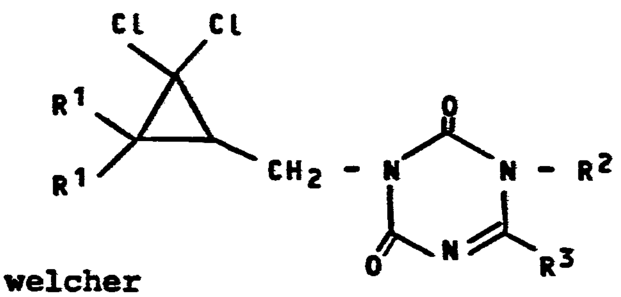

(I)

in welcher

$R^1$ für Wasserstoff oder Alkyl steht,

$R^2$ für Alkyl, für Amino oder für einen Rest $-N{=}C{<}^{R^4}_{R^5}$ steht, wobei $R^4$ und $R^5$ unabhängig voneinander für Wasserstoff, für Alkyl oder für gegebenenfalls substituiertes Aryl stehen, oder gemeinsam für einen zweifach verknüpften Alkandiylrest stehen, und

$R^3$ für Alkylthio, Alkylamino oder Dialkylamino steht,

erhält, wenn man

(a) 1,3,5-Triazin-2,4-dione der Formel (II),

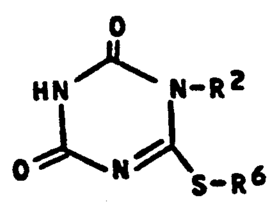

(II)

in welcher

$R^2$ die oben angegebene Bedeutung hat und

$R^6$ für Alkyl steht,

mit Cyclopropylmethylhalogeniden der Formel (III),

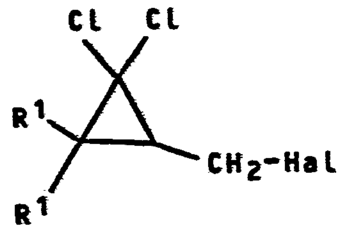

(III),

in welcher

$R^1$ die oben angegebene Bedeutung hat und

Hal für Halogen steht,

gegebenenfalls in Gegenwart eines Verdünnungsmittels und gegebenenfalls in Gegenwart eines Säurebindemittels umsetzt,

oder wenn man

(b) Isothioharnstoff-Derivate der Formel (IV),

$$H_2N - \underset{\displaystyle S - R^6}{\overset{}{C}} = N - R^7$$

(IV),

in welcher

$R^6$ die oben angegebene Bedeutung hat und

$R^7$ für Alkyl oder für gegebenenfalls substituiertes Alkylidenimino bzw. Cycloalkylidenimino steht,

oder deren Hydrosalze mit Cyclopropylmethylamin-Derivaten der Formel (V),

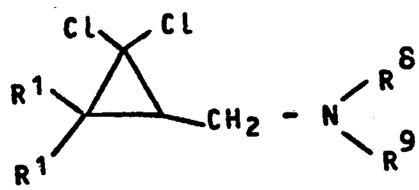

(V)

in welcher

$R^1$ die oben angegebene Bedeutung hat und

$R^8$ und $R^9$ unabhängig voneinander jeweils für gegebenenfalls substituiertes Aryloxycarbonyl stehen,

gegebenenfalls in Gegenwart eines Verdünnungsmittels und gegebenenfalls in Gegenwart eines Säurebindemittls umsetzt oder wenn man

(c) die nach Verfahren (a) oder (b) erhältlichen 1-Alkylidenimino-3-cyclopropylmethyl-1,3,5-triazin-2,4-dione der Formel (Ia),

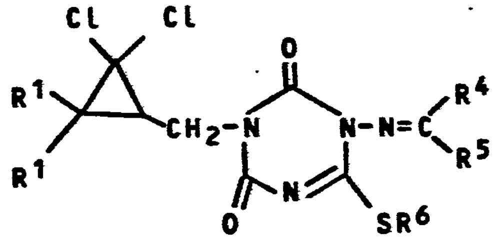

(Ia)

in welcher

$R^1$, $R^4$, $R^5$ und $R^6$ die oben angegebene Bedeutung haben,

entweder in prinzipiell bekannter Weise mit Säuren oder alternativ mit Isothiosemicarbaziden der Formel (IVa),

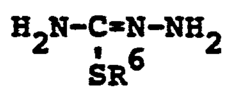

(IVa)

in welcher

$R^6$ die oben angegebene Bedeutung hat,

oder deren Hydrosalzen gegebenenfalls in Gegenwart eines Verdünnungsmittels und gegebenenfalls in Gegenwart eines Katalysators umsetzt zu den 1-Amino-3-cycloproyplmethyl-1,3,5-triazin-2,4-dionen der Formel (Ib),

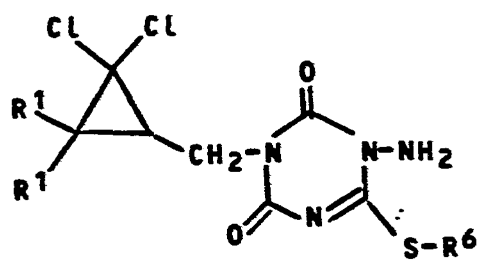

(Ib)

in welcher

$R^1$ und $R^6$ die oben angegebene Bedeutung haben,

oder wenn man

(d) die nach Verfahren (a), (b) oder (c) erhältlichen 6-Alkylthio-3-cyclopropylmethyl-1,3,5-triazin-2,4-dione der Formel (Ic),

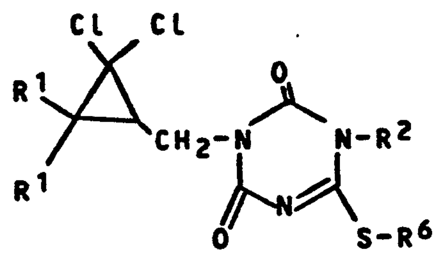

(Ic)

in welcher

$R^1$, $R^2$ und $R^6$ die oben angegebene Bedeutung haben,

mit Aminen der Formel (VI),

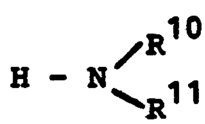

(VI)

in welcher

$R^{10}$ für Wasserstoff oder Alkyl steht und

$R^{11}$ für Alkyl steht,

gegebenenfalls in Gegenwart eines Verdünnungsmittels umsetzt zu den 6-Amino-3-cycloproyplmethyl-1,3,5-triazin-2,4-dionen der Formel (Id),

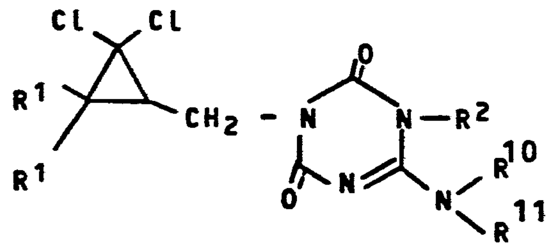

(Id)

in welcher

$R^1$, $R^2$, $R^{10}$ und $R^{11}$ die oben angegebene Bedeutung haben,

oder wenn man

(e) die nach Verfahren (a), (c) oder (d) erhältlichen 1-Amino-3-cyclopropylmethyl-1,3,5-triazin-2,4-dione der Formel (Ie),

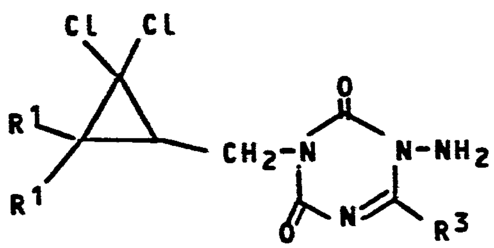

(Ie)

in welcher

$R^1$ und $R^3$ die oben angegebene Bedeutung haben,

mit Aldehyden oder Ketonen der Formel (VII),

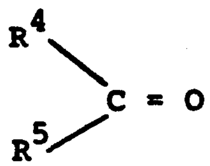

(VII)

in welcher

$R^4$ und $R^5$ die oben angegebene Bedeutung haben,

gegebenenfalls in Gegenwart eines Verdünnungsmittels und gegebenenfalls in Gegenwart eines Katalysators oder Reaktionshilfsmittels umsetzt zu den 1-Alkylidenimino-3-cyclopropylmethyl-1,3,5-triazin-2,4-dionen der Formel (If),

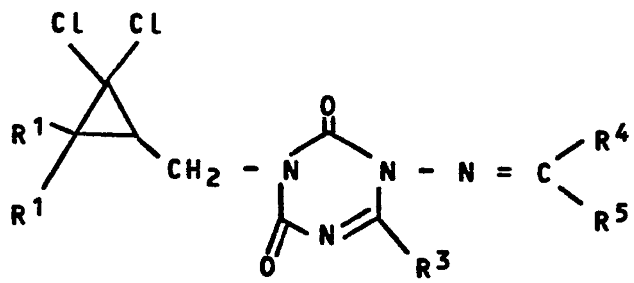

(If)

in welcher

$R^1$, $R^3$, $R^4$ und $R^5$ die oben angegebene Bedeutung haben.

Schließlich wurde gefunden, daß die neuen 3-Cyclopropylmethyl-1,3,5-triazin-2,4-dione der allgemeinen Formel (I) herbizide, insbesondere selektiv-herbizide Eigenschaften besitzen.

Überraschenderweise zeigen die erfindungsgemäßen 3-Cyclopropylmethyl-1,3,5-triazin-2,4-dione der Formel (I)

eine bessere herbizide Wirkung gegen Schadpflanzen und gleichzeitig eine verbesserte Verträglichkeit gegenüber wichtigen Kulturpflanzen im Vergleich zu den aus dem Stand der Technik bekannten 1,3,5-Triazin-2,4-dionen (wie beispielsweise 1-Amino-6-methylthio-3-phenyl-1,3,5-triazin-2,4-dion), welche chemisch und wirkungsmäßig naheliegende Verbindungen sind.

Die neuen 3-Cyclopropylmethyl-1,3,5-triazin-2,4-dione sind durch die Formel (I) allgemein definiert. Bevorzugt sind Verbindungen der Formel (I), bei welchen

$R^1$ für Wasserstoff oder für geradkettiges oder verzweigtes Alkyl mit bis zu 4 Kohlenstoffatomen steht,

$R^2$ für Amino, für geradkettiges oder verzweigtes Alkyl mit bis zu 4 Kohlenstoffatomen oder für einen Rest

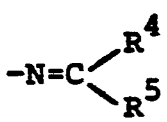 steht, wobei $R^4$ und $R^5$ jeweils unabhängig voneinander für Wasserstoff, für geradkettiges oder verzweigtes Alkyl mit bis zu 6 Kohlenstoffatomen oder für einfach oder mehrfach, gleich oder verschieden durch Halogen, Cyano, Nitro, sowie jeweils geradkettiges oder verzweigtes Alkyl, Alkoxy oder Alkylthio mit jeweils bis zu 4 Kohlenstoffatomen substituiertes Phenyl stehen oder gemeinsam für einen zweifach gebundenen Alkandiylrest mit 4 bis 6 Kohlenstoffatomen stehen und

$R^3$ für jeweils geradkettiges oder verzweigtes Alkylthio, Alkylamino oder Dialkylamino mit jeweils bis zu 6 Kohlenstoffatomen in den einzelnen Alkylteilen steht.

Besonders bevorzugt sind Verbindungen der Formel (I), bei welchen

$R^1$ für Wasserstoff, Methyl oder Ethyl steht,

$R^2$ für Methyl, Ethyl, Amino oder einen der Reste

$-N=C(CH_3)_2$, $-N=CH-C(CH_3)_3$, $-N=CH-C_6H_5$, -N=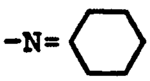 ,

-N=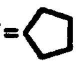 oder -N=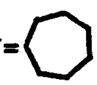 steht und

$R^3$ für Methylthio, Ethylthio, n- und i-Propylthio, n-, i-, s- und t-Butylthio, Methylamino, Ethylamino, Dimethylamino oder Diethylamino steht.

Im einzelnen seien die folgenden 3-Cyclopropylmethyl-1,3,5-triazin-2,4-dione der allgemeinen Formel (I) genannt:

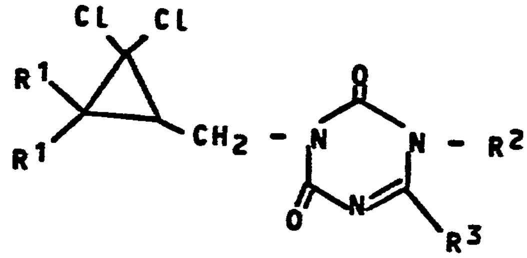

(I)

Tabelle 1

| $R^1$ | $R^2$ | $R^3$ |
|---|---|---|
| $CH_3$ | $CH_3$ | $-SCH_3$ |
| $CH_3$ | $CH_3$ | $-SC_2H_5$ |
| $CH_3$ | $CH_3$ | $-NH-CH_3$ |
| $CH_3$ | $CH_3$ | $-N(CH_3)_2$ |
| $CH_3$ | $CH_3$ | $-N(C_2H_5)_2$ |
| $CH_3$ | $C_2H_5$ | $-SCH_3$ |
| $CH_3$ | $C_2H_5$ | $-SC_2H_5$ |
| $CH_3$ | $C_2H_5$ | $-NH-CH_3$ |
| $CH_3$ | $C_2H_5$ | $-N(CH_3)_2$ |
| $CH_3$ | $C_2H_5$ | $-N(C_2H_5)_2$ |
| $CH_3$ | $NH_2$ | $-SCH_3$ |
| $CH_3$ | $NH_2$ | $-SC_2H_5$ |
| $CH_3$ | $NH_2$ | $-NH-CH_3$ |
| $CH_3$ | $NH_2$ | $-N(CH_3)_2$ |
| $CH_3$ | $NH_2$ | $-N(C_2H_5)_2$ |
| $CH_3$ | $-N=C(CH_3)_2$ | $-SCH_3$ |
| $CH_3$ | $-N=C(CH_3)_2$ | $-SC_2H_5$ |
| $CH_3$ | $-N=C(CH_3)_2$ | $-NH-CH_3$ |
| $CH_3$ | $-N=C(CH_3)_2$ | $-N(CH_3)_2$ |
| $CH_3$ | $-N=C(CH_3)_2$ | $-N(C_2H_5)_2$ |
| $CH_3$ | $-N=C(CH_3)_2$ | $-NH-C_2H_5$ |

Tabelle 1 (Fortsetzung)

| $R^1$ | $R^2$ | $R^3$ |
|---|---|---|
| $CH_3$ | $-N=CH-C_6H_5$ | $-SCH_3$ |
| $CH_3$ | $-N=CH-C_6H_5$ | $-SC_2H_5$ |
| $CH_3$ | $-N=CH-C_6H_5$ | $-NH-CH_3$ |
| $CH_3$ | $-N=CH-C_6H_5$ | $-N(CH_3)_2$ |
| $CH_3$ | $-N=CH-C_6H_5$ | $-N(C_2H_5)_2$ |
| $CH_3$ | $-N=CH-C_6H_5$ | $-NH-C_2H_5$ |
| $CH_3$ | -N= 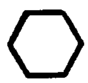 | $-SCH_3$ |
| $CH_3$ | -N= 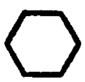 | $-SC_2H_5$ |
| $CH_3$ | -N= 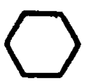 | $-NH-CH_3$ |
| $CH_3$ | -N= 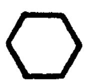 | $-NH-C_2H_5$ |
| $CH_3$ | -N= 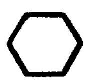 | $-N(CH_3)_2$ |
| $CH_3$ | -N= 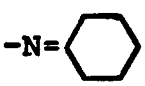 | $-N(C_2H_5)_2$ |
| H | $CH_3$ | $-SCH_3$ |
| H | $CH_3$ | $-SC_2H_5$ |
| H | $CH_3$ | $-NH-CH_3$ |
| H | $CH_3$ | $-N(CH_3)_2$ |
| H | $CH_3$ | $-N(C_2H_5)_2$ |
| H | $C_2H_5$ | $-SCH_3$ |
| H | $C_2H_5$ | $-SC_2H_5$ |
| H | $C_2H_5$ | $-NH-CH_3$ |
| H | $C_2H_5$ | $-N(CH_3)_2$ |
| H | $C_2H_5$ | $-N(C_2H_5)_2$ |

Tabelle 1 (Fortsetzung)

| $R^1$ | $R^2$ | $R^3$ |
|---|---|---|
| H | $NH_2$ | $-SCH_3$ |
| H | $NH_2$ | $-SC_2H_5$ |
| H | $NH_2$ | $-NH-CH_3$ |
| H | $NH_2$ | $-N(CH_3)_2$ |
| H | $NH_2$ | $-N(C_2H_5)_2$ |
| H | $-N=C(CH_3)_2$ | $-SCH_3$ |
| H | $-N=C(CH_3)_2$ | $-SC_2H_5$ |
| H | $-N=C(CH_3)_2$ | $-NH-CH_3$ |
| H | $-N=C(CH_3)_2$ | $-N(CH_3)_2$ |
| H | $-N=C(CH_3)_2$ | $-N(C_2H_5)_2$ |
| H | $-N=C(CH_3)_2$ | $-NH-C_2H_5$ |
| H | $-N=CH-C_6H_5$ | $-SCH_3$ |
| H | $-N=CH-C_6H_5$ | $-SC_2H_5$ |
| H | $-N=CH-C_6H_5$ | $-NH-CH_3$ |
| H | $-N=CH-C_6H_5$ | $-N(CH_3)_2$ |
| H | $-N=CH-C_6H_5$ | $-N(C_2H_5)_2$ |
| H | -N= 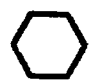 | $-SCH_3$ |
| H | -N= 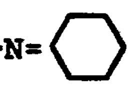 | $-SC_2H_5$ |
| H | -N= 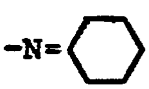 | $-NH-CH_3$ |
| H | -N= 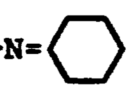 | $-NH-C_2H_5$ |
| H | -N= 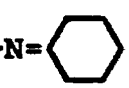 | $-N(CH_3)_2$ |
| H | -N= 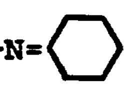 | $-N(C_2H_5)_2$ |

Verwendet man als Ausgangsstoffe beispielsweise 1-Methyl-6-methylthio-1,2,3,4-tetrahydro-1,3,5-triazin-2,4-dion und 1,1-Dichlor-2-chlormethyl-cyclopropan, so läßt sich der Reaktionsablauf des erfindungsgemäßen Verfahrens (a) durch das folgende Formelschema darstellen:

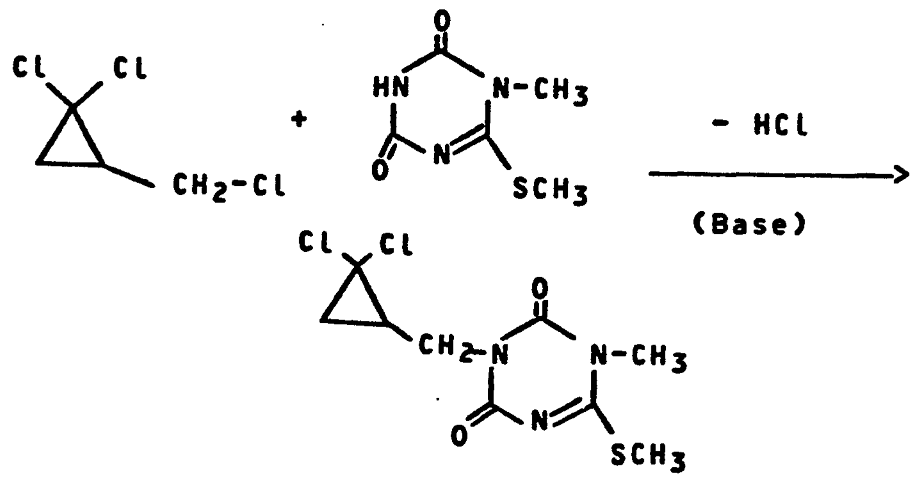


Verwendet man als Ausgangsstoffe beispielsweise Aceton-(S-methyl-isothiosemicarbazon) und N,N-Bisphenoxycarbonyl-1,1-dichlor-2-aminomethyl-cyclopropan, so läßt sich der Reaktionsablauf des erfindungsgemäßen Verfahrens (b) durch das folgende Formelschema darstellen:

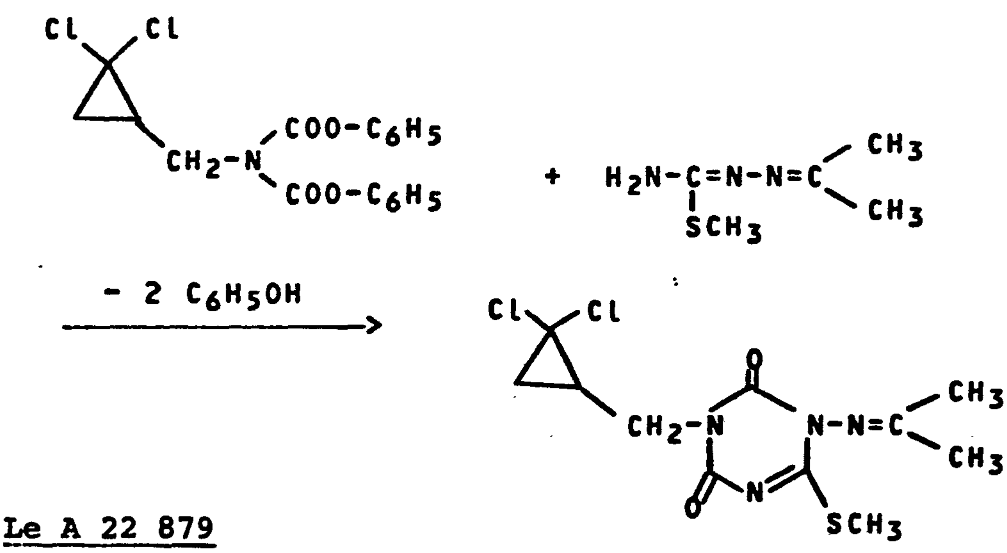

Verwendet man als Ausgangsstoffe beispielsweise 3-(2,2-Dichlorcyclopropylmethyl)-6-methylthio-1-phenylmethylidenimino-1,2,3,4-tetrahydro-1,3,5-triazin-2,4-dion und S-Methylisothiosemicarbazid, so läßt sich der Reaktionsablauf des erfindungsgemäßen Verfahrens (c) durch das folgende Formelschema darstellen:

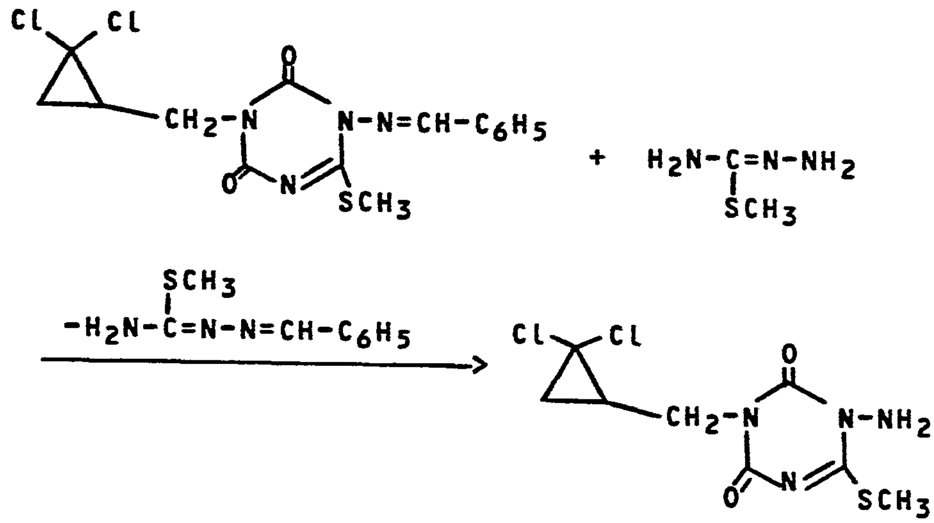

Verwendet man als Ausgangsstoffe beispielsweise 3-(2,2-Dichlorcyclopropylmethyl)-3-ethylthio-1-methyl-1,2,3,4-tetrahydro-1,3,5-triazin-2,4-dion und Dimethylamin, so läßt sich der Reaktionsablauf des erfindungsgemäßen Verfahrens (d) durch das folgende Formelschema darstellen

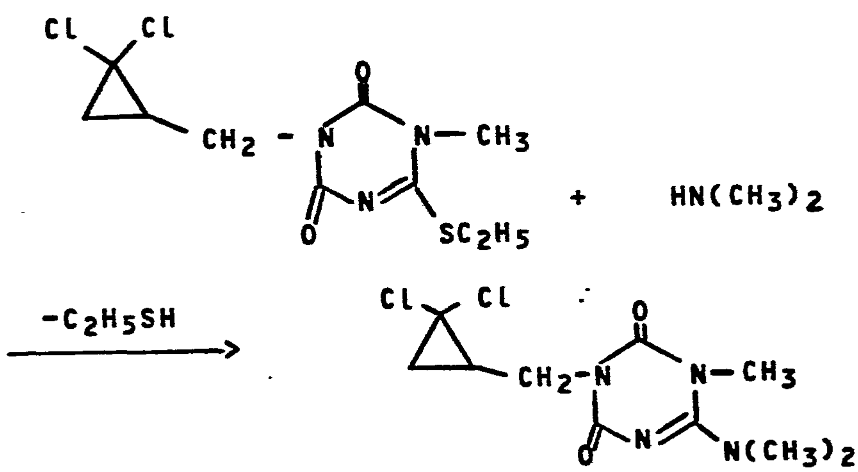

Verwendet man als Ausgangsstoffe beispielsweise 1-Amino-3-(2,2-dichlor-3,3-dimethyl-cyclopropylmethyl)-6-diethylamino-1,2,3,4-tetrahydro-1,3,5-triazin-2,4-dion und 4-Chlorbenzaldehyd, so läßt sich der Reaktionsablauf des erfindungsgemäßen Verfahrens (e) durch das folgende Formelschema darstellen:

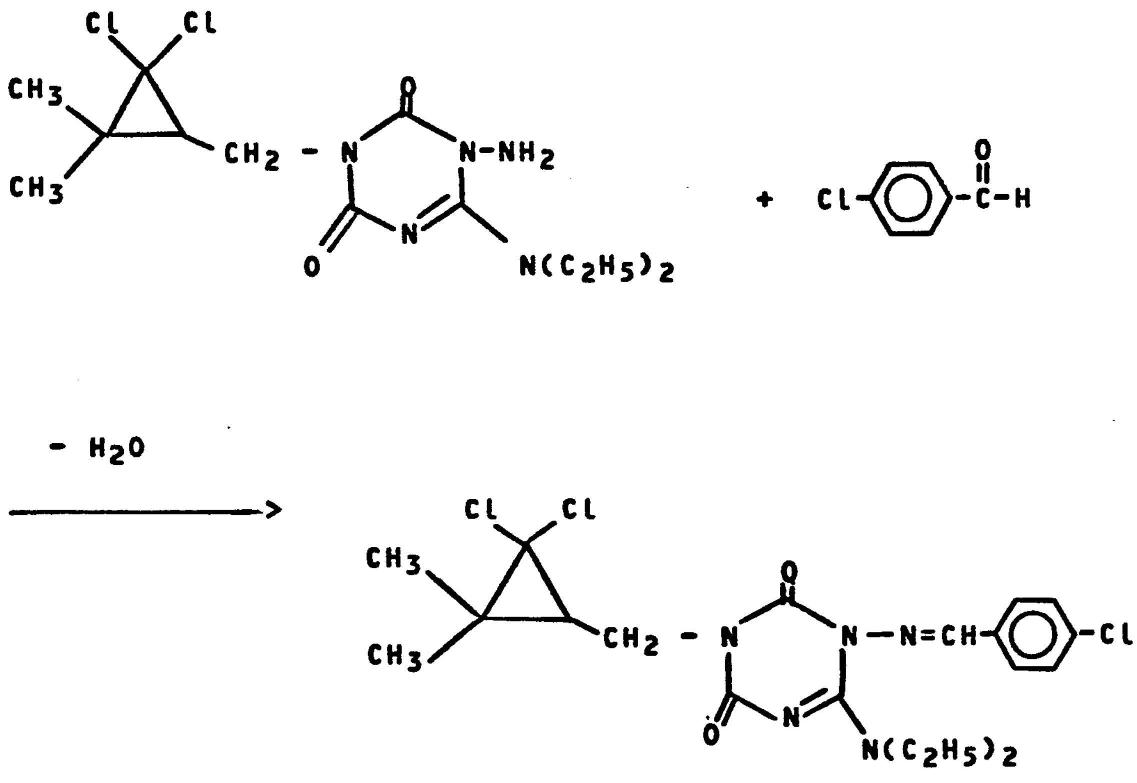

Die zur Durchführung des erfindungsgemäßen Verfahrens (a) als Ausgangsstoffe benötigten 1,3,5-Triazin-2,4-dione sind durch die Formel (II) allgemein definiert. In dieser Formel (II) steht $R^2$ vorzugsweise für diejenigen Reste, die schon bei der Beschreibung der erfindungsgemäßen Stoffe der Formel (I) vorzugsweise für diesen Substituenten genannt wurden. $R^6$ steht vorzugsweise für geradkettiges oder verzweigtes Alkyl mit bis zu 6 Kohlenstoffatomen.

Die 1,3,5-Triazin-2,4-dione der Formel (II) sind noch nicht bekannt. Diese neuen und wertvollen Zwischenprodukte (II) sind ebenfalls Gegenstand der Erfindung.

Man erhält die Verbindungen der Formel (II), wenn man Isothioharnstoff-Derivate der Formel (IV)

$$H_2N - \underset{\displaystyle SR^6}{C} = N - R^7 \qquad (IV)$$

in welcher

$R^6$ und $R^7$ die oben angegebenen Bedeutungen haben,

entweder mit Phenoxycarbonylisocyanat der Formel (VIII)

$$C_6H_5 - O - CO - NCO \qquad (VIII)$$

oder mit N,N-Bisphenoxycarbonylamin der Formel (IX),

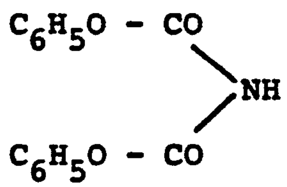

(IX)

gegebenenfalls in Gegenwart eines Verdünnungsmittels, wie beispielsweise Toluol, bei Temperaturen zwischen -20°C und +130°C umsetzt, zu den 1,3,5-Triazin-2,4-dionen der Formel (IIa)

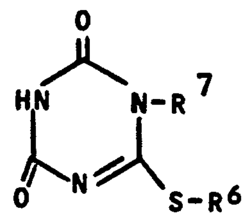

(IIa)

in welcher

$R^6$ und $R^7$ die oben angegebene Bedeutung haben,

und gegebenenfalls anschließend die derart erhältlichen 1,3,5-Triazin-2,4-dione der Formel (IIb),

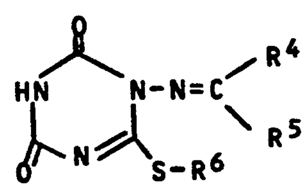

(IIb)

in welcher

$R^6$ die oben angegebene Bedeutung hat und

$R^4$ und $R^5$ unabhängig voneinander für Wasserstoff, für Alkyl oder für gegebenenfalls substituiertes Aryl stehen oder gemeinsam für einen zweifach verknüpften Alkandiylrest stehen, wobei der Rest

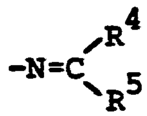 insbesondere für eine der Gruppen

$-N{=}C(CH_3)_2$, $-N{=}CH{-}C(CH_3)_3$, $-N{=}CH{-}C_6H_5$ oder

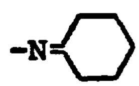 steht,

entweder in prinzipiell bekannter Weise mit wäßrigen Säuren, wie beispielsweise Salzsäure oder p-Toluolsulfonsäure, oder mit Isothiosemicarbaziden der Formel (IVa),

$$H_2N - \underset{\displaystyle SR^6}{\overset{}{C}} = N - NH_2 \qquad \text{(IVa)}$$

in welcher

$R^6$ die oben angegebene Bedeutung hat,

gegebenenfalls in Gegenwart eines Verdünnungsmittels, wie beispielsweise Isopropanol, bei Temperaturen zwischen +20°C und 120°C umsetzt zu den 1-Amino-1,3,5-triazin-2,4-dionen der Formel (IIc),

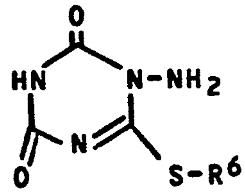

(IIc)

in welcher

$R^6$ die oben angegebene Bedeutung hat.

1,3,5-Triazin-2,4-dione der oben angegebenen Formel (IIb) erhält man alternativ, wenn man die oben beschriebene Umsetzung der Isothioharnstoff-Derivate der Formel (IV) mit Phenoxycarbonylisocyanat der Formel (VIII) oder mit N,N-Bisphenoxycarbonylamin der Formel (IX) in zwei Stufen durchführt, wobei man das intermediär auftretende Zwischenprodukt, welches die Struktur eines der beiden Isothioharnstoffisomeren der Formeln (Xa) und (Xb)

$$C_6H_5O\text{-}CO\text{-}NH\text{-}CO\text{-}N{=}\underset{}{\overset{SR^6}{C}}\text{-}NH\text{-}R^7 \qquad (Xa)$$

$$HN{=}\overset{SR^6}{C}\text{-}\underset{CO\text{-}NH\text{-}CO\text{-}O\text{-}C_6H_5}{N}\text{-}R^7 \qquad (Xb)$$

in welchen

$R^6$ und $R^7$ die oben angegebenen Bedeutungen haben,

besitzt,

bei Temperaturen unter +100°C isoliert und in getrennter Reaktion gegebenenfalls in Gegenwart eines Verdünnungsmittels wie beispielsweise Toluol bei Temperaturen zwischen +100°C und +130°C cyclisiert.

Die weiterhin zur Durchführung des erfindungsgemäßen Verfahrens (a) als Ausgangsstoffe benötigten Cyclopropylmethylhalogenide sind durch die Formel (III) allgemein definiert. In dieser Formel (III) steht $R^1$ vorzugsweise für diejenigen Reste, die schon bei der Beschreibung der erfindungsgemäßen Stoffe der Formel (I) als bevorzugt für diesen Substituenten genannt wurden. Hal steht vorzugsweise für Chlor oder Brom. Die Cyclopropylmethylhalogenide der Formel (III) sind bekannt (vgl. z.B. Acta Chem. Scand. Ser. B, <u>31</u>, 463-468 [1977]; Liebigs Ann. Chem. <u>1979</u>, 920-922; Tetrahedron Letters <u>24</u>, 1065-1066 [1983])oder lassen sich nach prinzipiell bekannten Methoden in analoger Weise leicht herstellen.

Die zur Durchführung des erfindungsgemäßen Verfahrens (b) als Ausgangsstoffe benötigten Isothioharnstoff-Derivate sind durch die Formel (IV) allgemein definiert. In dieser Formel (IV) steht $R^6$ vorzugsweise für Alkyl mit 1-6 C-Atomen; $R^7$ steht vorzugsweise für Alkyl mit 1-4 C-Atomen, Alkylidenimino mit 1-2 C-Atomen in jeder Alkylgruppe oder Cycloalkylidenimino mit 5-7 C-Atomen, insbesondere für Methyl, Ethyl oder einen der Reste $-N=C(CH_3)_2$, $-N=CH-C_6H_5$, $-N=CH-C(CH_3)_3$ oder -N= 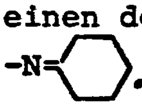.

Die Isothioharnstoff-Derivate der Formel (IV) sind bekannt (vgl. z.B. DE-OS 31 47 735).

Die weiterhin zur Durchführung des erfindungsgemäßen Verfahrens (b) als Ausgangsstoffe benötigten Cyclopropylmethylamin-Derivate sind durch die Formel (V) allgemein definiert. In dieser Formel (V) steht $R^1$ vorzugsweise für diejenigen Substituenten, die bereits bei der Beschreibung der erfindungsgemäßen Stoffe der Formel (I) als bevorzugt für diesen Rest genannt wurden.

$R^8$ und $R^9$ stehen vorzugsweise gleichzeitig für Phenoxycarbonyl.

Die Cyclopropylmethylamin-Derivate der Formel (V) sind noch nicht bekannt.

Man erhält sie, wenn man Cyclopropylmethylamine der Formel (XI)

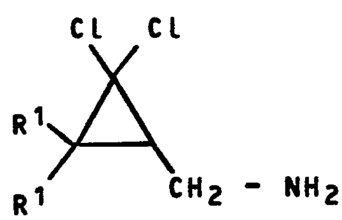

(XI)

in welcher

$R^1$ die oben angegebene Bedeutung hat,

mit allgemein bekannten Chlorameisensäurearylestern der Formel (XIIa)

$$R^8 - Cl \qquad \text{(XIIa)}$$

in welcher

$R^8$ die oben angegebene Bedeutung hat,

zunächst in einer ersten Stufe, gegebenenfalls in Gegenwart eines Verdünnungsmittels wie beispielsweise Methylenchlorid, und gegebenenfalls in Gegenwart eines Säurebindemittels, wie beispielsweise Natriumhydroxid, bei Temperaturen zwischen -20°C und +30°C umsetzt und die so erhaltenen monoacylierten Cyclopropylmethylamine der Formel (XIII)

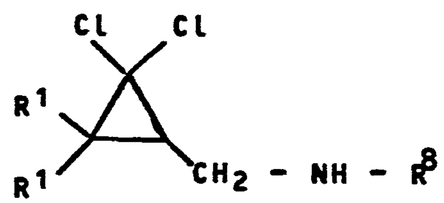

(XIII)

in welcher

$R^1$ und $R^8$ die oben angegebene Bedeutung haben,

in einer zweiten Stufe mit allgemein bekannten Chlorameisensäureestern der Formel (XIIb)

$$R^9 - Cl \qquad (XIIb)$$

in welcher

$R^9$ die oben angebenene Bedeutung hat,

gegebenenfalls in Gegenwart eines Verdünnungsmittels wie beispielsweise Methylenchlorid bei Temperaturen zwischen +80°C und +200°C umsetzt.

Die zur Durchführung des erfindungsgemäßen Verfahrens (c) als Ausgangsstoffe benötigten 1-Alkylidenimino-3-cyclopropylmethyl-1,3,5-triazin-2,4-dione sind durch die Formel (Ia) allgemein definiert. In dieser Formel (Ia) stehen $R^1$, $R^4$, $R^5$ und $R^6$ vorzugsweise für diejenigen Reste, die schon bei der Beschreibung der erfindungsgemäßen Stoffe der Formel (I) und der Vorprodukte der Formel (IIb) als bevorzugt für diese Substituenten genannt wurden.

Die 1-Alkylidenimino-3-cyclopropylmethyl-1,3,5-triazin-2,4-dione der Formel (Ia) sind erfindungsgemäße Verbindungen und erhältlich mit Hilfe der erfindungsgemäßen Verfahren (a) oder (b).

Die weiterhin zur Durchführung des erfindungsgemäßen Verfahrens (c) als Augangsstoffe benötigten Isothiosemicarbazide sind durch die Formel (IVa) allgemein definiert. In dieser Formel (IVa) steht $R^6$ vorzugsweise für diejenigen Substituenten, die schon bei der Beschreibung der erfindungsgemäßen Stoffe der Formel (I) als bevorzugt für diesen Rest genannt wurden.

Die Isothiosemicarbazide der Formel (IVa) sind allgemein bekannte Verbindungen der organischen Chemie.

Die zur Durchführung des erfindungsgemäßen Verfahrens (d) als Ausgangsstoffe benötigten 6-Alkylthio-3-cyclopropylmethyl-1,3,5-triazin-2,4-dione sind durch die Formel (Ic) allgemein definiert. In dieser Formel (Ic) stehen $R^1$, $R^2$ und $R^6$ vorzugsweise für diejenigen Reste, die schon bei der Beschreibung der erfindungsgemäßen Stoffe der Formel (I) und der Vorprodukte der Formel (II) vorzugsweise für diese Substituenten genannt wurden.

Die 6-Alkylthio-3-cyclopropylmethyl-1,3,5-triazin-2,4-dione sind erfindungsgemäße Verbindungen und erhältlich mit Hilfe der erfindungsgemäßen Verfahren (a), (b) oder (c).

Die weiterhin zur Durchführung des erfindungsgemäßen Verfahrens (d) als Ausgangsstoffe benötigten Amine sind durch die Formel (VI) allgemein definiert. In dieser Formel (VI) stehen $R^{10}$ und $R^{11}$ unabhängig voneinander vorzugsweise für Wasserstoff oder für geradkettiges oder verzweigtes Alkyl mit bis zu 6 Kohlenstoffatomen, insbesondere für Methyl oder Ethyl.

Die Amine der Formel (VI) sind allgemein bekannte Verbindungen der organischen Chemie.

Die zur Durchführung des erfindungsgemäßen Verfahrens (e) als Ausgangsstoffe benötigten 1-Amino-3-cyclopropylmethyl-1,3,5-triazin-2,4-dione sind durch die Formel (Ie) allgemein definiert. In dieser Formel stehen $R^1$ und $R^3$ vorzugsweise für diejenigen Reste, die bereits bei der Beschreibung der erfindungsgemäßen Stoffe der Formel (I) als bevorzugt für diese Substituenten genannt wurden. Die 1-Amino-3-cyclopropylmethyl-1,3,5-triazin-2,4-dione der Formel (Ie) sind erfindungsgemäße Stoffe und erhältlich mit Hilfe der erfindungsgemäßen Verfahren (a), (c) und (d).

Die weiterhin zur Durchführung des erfindungsgemäßen Verfahrens (e) als Ausgangsstoffe benötigten Aldehyde und Ketone sind durch die Formel (VII) allgemein definiert. In dieser Formel (VII) stehen $R^4$ und $R^5$ vorzugsweise für diejenigen Substituenten, die schon bei der Herstellung der erfindungsgemäßen Stoffe der Formel (I) und der Vorprodukte (IV) als bevorzugt für diese Reste genannt wurden. Die Aldehyde und Ketone der Formel (VII) sind allgemein bekannte Verbindungen der organischen Chemie.

Die zur Herstellung der Vorprodukte der Formel (II) als Ausgangsstoffe benötigten Isothioharnstoff-Derivate der Formel (IV) sind bekannt (vgl. z.B. DE-OS 31 47 735 oder US-PS 4 053 299).

Das zur Herstellung der Vorprodukte der Formel (II) weiterhin als Ausgangsstoff benötigte Phenoxycarbonylisocyanat der Formel (VIII) ist ebenfalls bekannt (vgl. z.B. EP 55 442).

Das alternativ zur Herstellung der Vorprodukte der Formel (II) verwendbare N,N-Bisphenoxycarbonylamin der Formel (IX) ist noch nicht bekannt. Man erhält es, wenn man das bekannte Chlorcarbonylisocyanat der Formel (XIV),

$$Cl-CO-N=C=O \qquad (XIV)$$

(vgl. z.B. DE-OS 31 50 983 oder EP 55 430) mit Phenol, gegebenenfalls in Gegenwart eines Verdünnungsmittels wie beispielsweise Toluol, bei Temperaturen zwischen + 20°C und +150°C umsetzt.

Die zur Herstellung der Vorprodukte der Formel (V) als Ausgangsstoffe benötigten Cyclopropylmethylamine der Formel (XI) sind bekannt (vgl. z.B. DE-OS 28 15 689 oder Liebigs Annalen der Chemie 1979, 920-922) oder lassen sich nach prinzipiell bekannten Verfahren in analoger Weise herstellen.

Die weiterhin zur Herstellung der Vorprodukte der Formel (V) als Ausgangsstoffe benötigten Chlorameisensäurearylester der Formeln (XIIa) und (XIIb) sind allgemein bekannte Verbindungen der organischen Chemie.

Als Verdünnungsmittel zur Durchführung des erfindungsgemäßen Verfahrens (a) kommen alle inerten organischen Lösungsmittel in Frage. Vorzugsweise verwendet man dipolare aprotische Lösungsmittel, wie beispielsweise Acetonitril oder Propionitril, Dimethylformamid oder Dimethylacetamid, N-Methylpyrrolidon oder Hexamethylphosphorsäuretriamid, Dioxan oder Dimethylsulfoxid.

Als Säurebindemittel zur Durchführung des erfindungsgemäßen Verfahrens (a) kommen alle üblicherweise verwendbaren anorganischen oder organischen Basen in Frage. Vorzugsweise verwendet man Alkalimetallhydroxide, -carbonate oder -hydrogencarbonate wie beispielsweise Natriumhydroxid oder Kaliumhydroxid, Natriumcarbonat, Kaliumcarbonat oder Natriumhydrogencarbonat oder tertiäre Amine wie Triethylamin, N,N-Dimethylanilin, 4-Dimethylaminopyridin, Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man zwischen -20°C und +120°C, vorzugsweise zwischen 0°C und +80°C.

Zur Durchführung des erfindungsgemäßen Verfahrens (a) setzt man pro Mol 1,3,5-Triazin-2,4-dion der Formel (II) im allgemeinen 1,0 bis 1,5 Mol, vorzugsweise äquimolare Mengen an Cyclopropylmethylhalogenid der Formel (III) und im allgemeinen 1,0 bis 1,5 Mol, vorzugsweise äquimolare Mengen an Säurebindemittel ein. Die Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen ebenfalls inerte organische Lösungsmittel in Frage. Vorzugsweise verwendet man aliphatische oder aromatische, gegebenenfalls halogenierte Kohlenwasserstoffe, wie beispielsweise Benzin, Benzol, Toluol, Xylol oder Chlorbenzol, Methylenchlorid, Chloroform oder Tetrachlorkohlenstoff oder Alkohole, wie Methanol, Ethanol, n- und i-Propanol oder n- und i-Butanol. Man kann jedoch auch ohne Verwendung von Lösungsmitteln in der Schmelze der Ausgangsprodukte arbeiten.

Als Säurebindemittel kommen bei der Durchführung des erfindungsgemäßen Verfahrens (b) alle üblicherweise verwendbaren organischen oder anorganischen Basen in Frage. Vorzugsweise verwendet man die bei Verfahren (a) genannten Säurebindemittel.

Die Reaktionstemperaturen können bei der Durchführung des erfindungsgemäßen Verfahrens (b) ebenfalls in einem größeren Bereich variiert werden. Im allgemeinen arbeitet man zwischen +20°C und +200°C, vorzugsweise zwischen +50°C und +150°C.

Zur Durchführung des erfindungsgemäßen Verfahrens (b) setzt man pro Mol Isothioharnstoff-Derivat der Formel (IV) im allgemeinen 0,8 bis 1,5 Mol, vorzugsweise äquimolare Mengen an Cyclopropylmethylamin-Derivaten der Formel (V) ein und bei Verwendung von Isothioharnstoff-Hydrosalzen als Ausgangsstoffe im allgemeinen 0,8 bis 1,5 Mol, vorzugsweise äquimolare Mengen an Säurebindemittel ein. Das bei dieser Cyclisierungsreaktion freiwerdende gegebenenfalls substituierte Phenol wird entweder im Vakuum abdestilliert oder extraktiv durch Behandlung mit wäßrigen Basen, wie beispielsweise Natronlauge entfernt. Die Aufarbeitung und Isolierung der Reaktionsprodukte der Formel (Ia) erfolgt nach üblichen Methoden.

Als Verdünnungsmittel zur Durchführung des erfindungsgemäßen Verfahrens (c) kommen ebenfalls inerte organische Lösungsmittel in Frage; vorzugsweise verwendet man Kohlenwasserstoffe wie Benzin, Benzol, Toluol oder Xylol oder Alkohole wie Methanol, Ethanol, Propanol oder Butanol.

Als Spaltungsreagenz ebenso wie als Katalysator kommen anorganische oder organische Säuren in Frage. Vorzugsweise verwendet man Mineralsäuren wie beispielsweise Salzsäure oder Schwefelsäure oder organische Sulfonsäuren wie beispielsweise p-Toluolsulfonsäure.

Die Reaktionstemperaturen können bei der Durchführung des erfindungsgemäßen Verfahrens (c) ebenfalls in einem größeren Bereich variiert werden. Im allgemeinen arbeitet man zwischen +20°C und +150°C, vorzugsweise zwischen +40°C und +120°C.

Zur Durchführung des erfindungsgemäßen Verfahrens (c) setzt man pro Mol 1-Alkylidenimino-3-cyclopropylmethyl-1,3,5-triazin-2,4-dion der Formel (Ia) 0,01 bis 0,5 Mol an anorganischer oder organischer Säure und gegebenenfalls 1,0 bis 1,5 Mol an Isothiosemicarbazid der Formel (IVa) ein. Die Aufarbeitung und Isolierung der Endprodukte der Formel (Ib) erfolgt nach bekannten und üblichen Verfahren (vgl. z.B. DE-OS 22 54 200, US-PS 4 056 527, DE-OS 31 47 735, DE-OS 32 41 114).

Als Verdünnungsmittel zur Durchführung des erfindungsgemäßen Verfahrens (d) kommen wäßrige oder anorganische Lösungsmittel in Frage. Vorzugsweise verwendet man Alkohole, Wasser oder Wasser-Alkohol-Gemische, wobei als Alkohole die niedermolekularen Alkohole Methanol, Ethanol, Isopropanol oder n-Propanol bevorzugt sind.

Die Reaktionstemperaturen können bei der Durchführung des erfindungsgemäßen Verfahrens (d) in einem größeren Bereich variiert werden. Im allgemeinen arbeitet man zwischen +20°C und 150°C, vorzugsweise zwischen +40°C und +120°C.

Zur Durchführung des erfindungsgemäßen Verfahrens (d) setzt man pro Mol 6-Alkylthio-3-cyclopropylmethyl-1,3,5-triazin-2,4-dion der Formel (Ic) im allgemeinen 1 bis 10 Mol, vorzugsweise 1 bis 7 Mol an Amin der Formel (VI) ein. Die Aufarbeitung erfolgt nach allgemein üblichen Verfahren.

Als Verdünnungsmittel zur Durchführung des erfindungsgemäßen Verfahrens (e) kommen ebenfalls inerte organische Lösungsmittel in Frage. Vorzugsweise verwendet man die bei Verfahren (c) aufgezählten Lösungsmittel.

Als Katalysator zur Durchführung des erfindungsgemäßen Verfahrens (e) kommen insbesondere Säuren in Frage. Vorzugsweise verwendet man anorganische Mineralsäuren, wie beispielsweise Schwefelsäure oder Salzsäure oder organische Sulfonsäuren, wie beispielsweise p-Toluolsulfonsäure.

Die Reaktionstemperaturen können bei der Durchführung des erfindungsgemäßen Verfahrens (e) ebenfalls in einem größeren Bereich variiert werden. Im allgemeinen arbeitet man zwischen +20°C und +150°C, vorzugsweise zwischen +40°C und +120°C.

Bei der Durchführung des erfindungsgemäßen Verfahrens (e) setzt man pro Mol 1-Amino-3-cyclopropylmethyl-1,3,5-triazin-2,4-dion der Formel (Ie) im allgemeinen 1,0 bis 1,5 Mol, vorzugsweise 1,0 bis 1,3 Mol an Aldehyd oder Keton der Formel (VII) und 0,01 bis 0,5 Mol an Katalysator ein. Die Aufarbeitung und Isolierung der Endprodukte der Formel (If) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Verbindungen der Formel (I) neben einer deutlich besseren herbiziden Wirkung gegenüber wichtigen Schadpflanzen auch eine erheblich bessere Verträglichkeit gegenüber wichtigen Kulturpflanzen als die aus dem Stand der Technik bekannten Verbindungen und können zur selektiven Unkrautbekämpfung sowohl in dikotylen Kulturen wie z.B. Baumwolle, als auch in monokotylen Kulturen wie z.B. Hafer oder Mais eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen

Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage:

z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der

Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

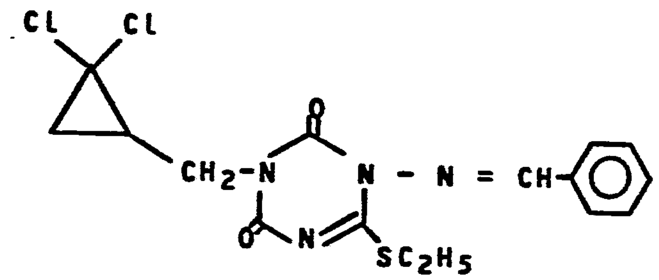

(Verfahren a)

1932 g (7 Mol) 1-Benzylidenimino-6-ethylthio-1,2,3,4-tetrahydro-1,3,5-triazin-2,4-dion und 1428 g (7 Mol) 1,1-Dichlor-2-brommethylcyclopropan in 5 Liter Dimethylsulfoxid werden bei 50°C unter Rühren portionsweise mit 966 g (7 Mol) gemahlenem Kaliumcarbonat versetzt. Nach beendeter Zugabe rührt man weitere 20 Stunden bei 50°C, gießt den Ansatz in 8 Liter Wasser und extrahiert mehrfach mit Dichlormethan. Die vereinigten organischen Phasen werden viermal mit Wasser und einmal mit gesättigter Natriumchloridlösung gewaschen, über Natriumsulfat getrocknet und im Vakuum eingeengt. Man erhält 2691 g (96,3 % der Theorie) an 1-Benzylidenimino-3-(2,2-dichlorcyclopropylmethyl)-6-ethylthio-1,2,3,4-tetrahydro-1,3,5-triazin-2,4-dion vom Schmelzpunkt 118°C.

(Verfahren e) (Beispiel 1; alternative Herstellung)

Eine Lösung von 15,6 g (0,05 Mol) 1-Amino-3-(2,2-dichlorcyclopropylmethyl)-6-ethylthio-1,2,3,4-tetrahydro-1,3,5-triazin-2,4-dion in 250 ml Isopropanol wird nach Zugabe von 5,9 g (0,055 Mol) frisch destilliertem Benzaldehyd und 0,2 g p-Toluolsulfonsäurehydrat 1 Stunde unter Rückfluß gekocht. Die erhaltene Reaktionsmischung wird bei 0°C abgesaugt und das Produkt mit kaltem Isopropanol nachgewaschen. Man erhält 17,0 g (85 % der Theorie) an 1-Benzylidenimino-3-(2,2-dichlorcyclopropylmethyl)-6-ethylthio-1,2,3,4-tetrahydro-1,3,5-triazin-2,4-dion, welches nach Umkristallisation aus Methanol bei 110°C schmilzt.

Beispiel 2

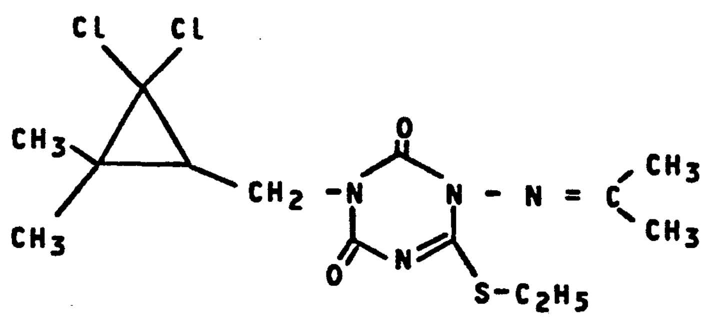

(Verfahren a)

5 g (0,022 Mol) 6-Ethylthio-1-isopropylidenimino-1,2,3,4-tetrahydro-1,3,5-triazin-2,4-dion, 5,1 g (0,022 Mol) 1,1-Dichlor-2,2-dimethyl-3-brommethylcyclopropan und

3,1 g (0,022 Mol) Kaliumcarbonat werden in 30 ml Dimethylsulfoxid 14 Stunden bei Raumtemperatur gerührt. Zur Aufarbeitung gießt man den Ansatz in 100 ml Wasser, extrahiert mehrfach mit Dichlormethan, wäscht die vereinigten organischen Phasen fünfmal mit Wasser, trocknet über Natriumsulfat und entfernt das Lösungsmittel im Vakuum. Das verbleibende Oel kristallisiert aus Ligroin. Man erhält 7,7 g (92,3 % der Theorie) an 3-(2,2-Dichlor-3,3-dimethyl-cyclopropylmethyl)-6-ethylthio-1-isopropylidenimino-1,2,3,4-tetrahydro-1,3,5-triazin-2,4-dion vom Schmelzpunkt 89-90°C.

Beispiel 3

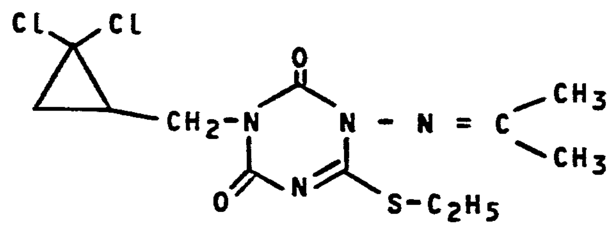

(Verfahren b)

Zu einer Schmelze von 304 g (0,8 Mol) N,N-Bisphenoxycarbonyl-(2,2-dichlorcyclopropylmethyl)-amin gibt man bei 80°C 151,1 g (0,95 Mol) S-Ethyl-1-isopropyliden-isothiosemicarbazid und rührt nach beendeter Zugabe weitere 5 Stunden bei 100°C. Zur Aufarbeitung destilliert man die Hauptmenge des gebildeten Phenols im Vakuum ab und

löst den harzartigen Rückstand in siedendem Methanol, aus welchem das Produkt beim Erkalten auskristallisiert. Man erhält 189,8 g (67,6 % der Theorie) an 3-(2,2-Dichlorcyclopropylmethyl)-6-ethylthio-1-isopropylidenimino-1,2,3,4-tetrahydro-1,3,5-triazin-2,4-dion vom Schmelzpunkt 102°C-103°C.

Herstellung der Ausgangsverbindungen

a)

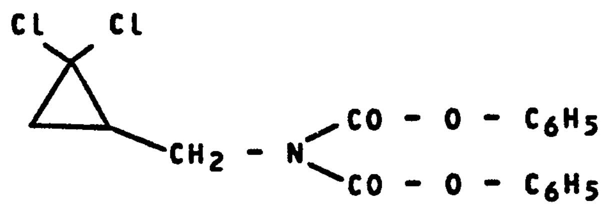

Eine Lösung von 780 g (3 Mol) N-(2,2-Dichlorcyclopropylmethyl)-carbamidsäure-phenylester in 700 ml Dichlormethan tropft man innerhalb von 8 Stunden gleichmäßig unter die Oberfläche von 3800 ml (30 Mol) siedendem Chlorameisensäurephenylester (Kp: 185-187°C), wobei gleichzeitig ein trockener Stickstoffstrom durch die Lösung geleitet wird. Das Dichlormethan wird dabei über den Kopf eines auf 100°C temperierten Rückflußkühlers abdestilliert. Nach beendeter Zugabe rührt man weitere 2 Stunden bei einer Sumpftemperatur von 187°C bis 190°C unter gleichzeitigem Durchleiten von Stickstoff, destilliert dann überschüssigen Chlorameisensäurephenylester im Wasserstrahlvakuum ab und destilliert den Rückstand an einer kurzen Füllkörperkolonne im Hochvakuum an, bis ein Siedepunkt von 95°C bei 0,17 mbar erreicht

ist. Als Rückstand verbleiben 1137 g Produkt mit einem GC-Gehalt von 93 % (Ausbeute 92,7 % der Theorie), welches durch fraktionierte Destillation weiter gereinigt werden kann. Man erhält 1011 g (88,7 % der Theorie) an N,N-Bisphenoxycarbonyl-(2,2-dichlorcyclopropylmethyl)-amin vom Siedepunkt 178°C/0,005 mbar, welches aus Petroether kristallisiert. Schmelzpunkt 55°C-56°C.

b)

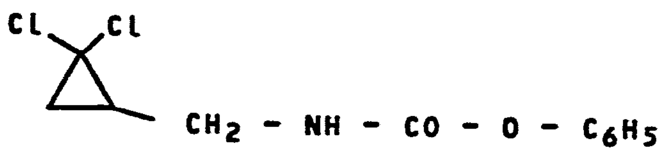

Zu einer Lösung von 632 g (4,04 Mol) Chlorameisensäurephenylester in 2 Liter Dichlormethan tropft man unter Kühlung bei 15°C bis 20°C gleichzeitig 453 ml (565,5 g bzw. 4,04 Mol) (2,2-Dichlorcyclopropylmethyl)-amin sowie 453 ml einer wäßrigen Natriumhydroxidlösung, die in dem angegebenen Volumen 162 g (4,04 Mol) Natriumhydroxid enthält. Nach beendeter Zugabe rührt man eine weitere Stunde bei Raumtemperatur, verdünnt mit 700 ml Wasser, trennt die organische Phase ab, trocknet über Natriumsulfat und entfernt das Lösungsmittel im Vakuum. Man erhält 1041 g (99,1 % der Theorie) an N-(2,2-Dichlorcyclopropylmethyl)-carbamidsäurephenylester, der sich aus Essigester/Petrolether umkristallisieren läßt. Schmelzpunkt 67°C-68°C.

Beispiel 4

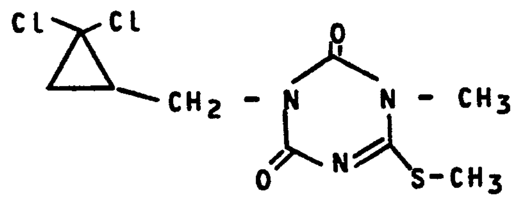

(Verfahren b)

Eine Mischung von 154,1 g (0,4 Mol) N,N-Bisphenoxycarbonyl-(2,2-dichlorcyclopropylmethyl)-amin, 92,8 g (0,4 Mol) N,S-Dimethyl-isothioharnstoff-hydroiodid und 40,4 g Triethylamin in 400 ml sec.-Butanol wird 5 Stunden bei 100°C gerührt. Zum erkalteten Reaktionsgemisch gibt man 250 ml Wasser, trennt die organische Phase ab, wäscht sie nochmals mit Wasser, trocknet über Natriumsulfat und destilliert im Vakuum das Lösungsmittel und die Hauptmenge des gebildeten Phenols ab. Der Rückstand wird aus Methanol umkristallisiert. Man erhält 74,4 g (62,8 % der Theorie) an 3-(2,2-Dichlor-cyclopropylmethyl)-1-methyl-6-methylthio-1,2,3,4-tetrahydro-1,3,5-triazin-2,4-dion vom Schmelzpunkt 107°C-109°C.

Beispiel 5

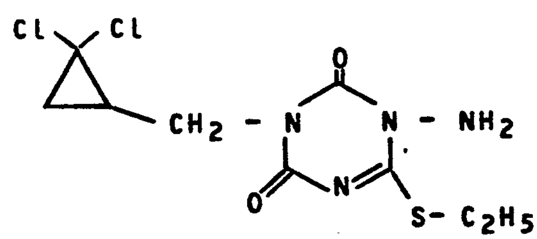

(Verfahren c)

Zu 178 g (0,507 Mol) 3-(2,2-Dichlorcyclopropylmethyl)-6-ethylthio-1-isopropylidenimino-1,2,3,4-tetrahydro-1,3,5-triazin-2,4-dion in 507 ml Isopropanol gibt man bei 60°C 4,8 g (0,025 Mol) p-Toluolsulfonsäurehydrat und 18,3 g Wasser und rührt eine Stunde bei 60°C, wobei das Produkt teilweise ausfällt. Die erhaltene Reaktionsmischung wird im Vakuum auf die Hälfte eingeengt und das kristalline Produkt bei 0°C abgesaugt. Man erhält 148,6 g (93,9 % der Theorie) an 1-Amino-3-(2,2-dichlorcyclopropylmethyl)-6-ethylthio-1,2,3,4-tetrahydro-1,3,5-triazin-2,4-dion vom Schmelzpunkt 183°C-185°C.

(Verfahren c) (Beispiel 5; alternative Herstellung)

Zu 637 g (7 Mol) Thiosemicarbazid in 4 Litern Toluol gibt man 1078 g (7 Mol) Diethylsulfat und rührt 4 Stunden bei 80°C. Man kühlt auf 50°C ab, gibt 2793 g (7 Mol) 1-Benzylidenimino-3-(2,2-dichlorcyclopropylmethyl)-6-ethylthio-1,2,3,4-tetrahydro-1,3,5-triazin-2,4-dion und 2 g p-Toluolsulfonsäure zu und rührt über Nacht bei 50°C. Die abgekühlte Reaktionsmischung wird bei 20°C mit einer Lösung von 280 g (7 Mol) Natriumhydroxid in 2 l Wasser versetzt, zwei Stunden bei Raumtemperatur gerührt, abgesaugt und getrocknet. Man erhält 1754 g (80,6 % der Theorie) an 1-Amino-3-(2,2-dichlorcyclopropylmethyl)-6-ethylthio-1,2,3,4-triazin-2,4-dion vom Schmelzpunkt 184°C-186°C.

**Beispiel 6**

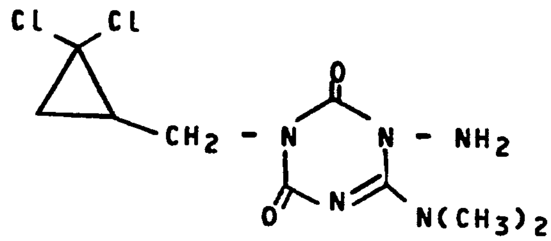

(Verfahren d)

Man rührt eine Suspension von 22,6 g (0,076 Mol) 1-Amino-3-(2,2-dichlorcyclopropylmethyl)-6-methylthio-1,2,3,4-tetrahydro-1,3,5-triazin-2,4-dion in 88,5 g (0,93 Mol) 47,5 %iger wäßriger Dimethylaminlösung 8 Stunden bei Raumtemperatur, gibt dann 35 ml Isopropanol zu und erhitzt bis zum Rückfluß. Anschließend destilliert man so viel Lösungsmittel ab, bis eine Sumpftemperatur von 80°C erreicht ist. Aus dem klaren Rückstand kristallisiert beim Erkalten das Produkt aus. Man erhält 20,8 g (93,1 % der Theorie) an 1-Amino-3-(2,2-dichlorcyclopropylmethyl)-6-dimethylamino-1,2,3,4-tetrahydro-1,3,5-triazin-2,4-dion vom Schmelzpunkt 192°C - 194°C.

In entsprechender Weise erhält man die folgenden Verbindungen der allgemeinen Formel (I):

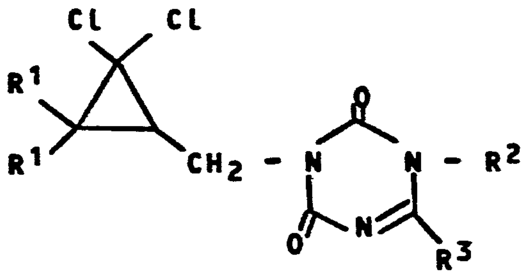

(I)

| Bsp. Nr. | $R^1$ | $R^2$ | $R^3$ | Schmelzpunkt (°C) |
|---|---|---|---|---|
| 7 | $CH_3$ | $CH_3$ | $CH_3S-$ | 124-126 |
| 8 | H | $(CH_3)_2C=N-$ | $CH_3S-$ | 50- 53 |
| 9 | H | $H_2N-$ | $CH_3S-$ | 210-214 |
| 10 | $CH_3$ | $H_2N-$ | $C_2H_5S-$ | 110-112 |
| 11 | $CH_3$ | $H_2N-$ | $CH_3S-$ | 144-148 |
| 12 | $CH_3$ | $(CH_3)_2C=N-$ | $CH_3S-$ | 108-110 |
| 13 | H | $H_2N-$ | $CH_3NH-$ | 226-228 |
| 14 | H | $H_2N-$ | $C_2H_5-NH-$ | 202-204 |
| 15 | H | $CH_3$ | $CH_3-NH-$ | 225-226 |
| 16 | H | $CH_3$ | $(CH_3)_2N-$ | 136-138 |
| 17 | $CH_3$ | $H_2N-$ | $(CH_3)_2N-$ | 103-106 |
| 18 | $CH_3$ | $H_2N-$ | $CH_3-NH-$ | 141-142 * |
| 19 | $CH_3$ | $CH_3$ | $(CH_3)_2N-$ | Öl |

* [Kristalle enthalten 1 Äquivalent Toluol]

0154906

Beispiel II-1

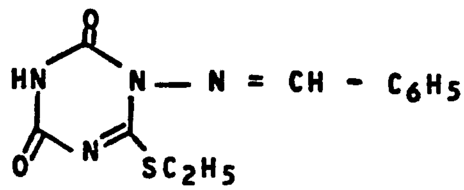

2313 g (9 Mol) N,N-Bisphenoxycarbonyl-amin und 1863 g (9 Mol) S-Ethyl-1-benzyliden-isothiosemicarbazid in 4 Liter Toluol werden 20 Stunden unter Rückfluß gekocht. Man läßt die Mischung auf 70°C abkühlen, gibt 2 Liter Essigester zu und rührt 5 Stunden, bis der Ansatz Raumtemperatur erreicht hat. Der ausgefallene Niederschlag wird abgesaugt, zweimal mit Essigester nachgewaschen und getrocknet. Man erhält 1907 g (77 % der Theorie) an 1-Benzylidenimino-6-ethylthio-1,2,3,4-tetrahydro-1,3,5-triazin-2,4-dion vom Schmelzpunkt 238°C.

Herstellung der Ausgangsverbindung:

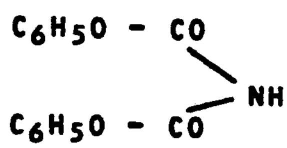

In eine Lösung von 949 g (10,1 Mol) Phenol in 5 Liter Toluol läßt man 378 ml (527,5 g/5,0 Mol) Chlorcarbonylisocyanat einfließen, wobei die Innentemperatur auf 60°C ansteigt und ab 50°C Salzsäuregas freigesetzt wird. Man

rührt bis zum Nachlassen der Gasentwicklung bei 60°C, erwärmt dann langsam auf Rückflußtemperatur und destilliert nach beendeter Gasentwicklung im Vakuum ca. 2 Liter Lösungsmittel ab. Das aus der erhaltenen Reaktionsmischung auskristallisierte Produkt wird bei 15°C abgesaugt, mit wenig kaltem Toluol gewaschen und bei 50°C im Vakuum getrocknet. Man erhält 1195 g (93 % der Theorie) an N,N-Bisphenoxycarbonyl-amin vom Schmelzpunkt 124°C bis 126°C.

<u>Beispiel II-2</u>:

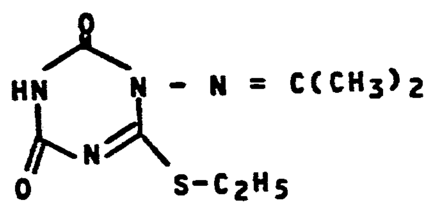

1200 g (3,73 Mol) S-Ethyl-1-isopropyliden-2-[bzw. 4]-phenoxy-carbamoylcarbonyl-isothiosemicarbazid werden in 3,6 Liter Toluol 4 Stunden unter Rückfluß gekocht. Aus dem erkalteten Gemisch saugt man 232 g Rohprodukt ab. Weitere 75 g Rohprodukt erhält man aus dem Eindampfrückstand der Mutterlauge nach Verrühren mit der doppelten Menge Isopropanol. Die vereinigten Rohprodukte werden aus Methanol umkristallisiert. Man erhält 295 g (34,7 % der Theorie) an 6-Ethylthio-1-isopropylidenimino-1,2,3,4-tetrahydro-1,3,5-triazin-2,4-dion vom Schmelzpunkt 195-197°C.

Herstellung der Ausgangsverbindung:

$$C_6H_5O\text{-}CO\text{-}NH\text{-}CO\text{-}N{=}\underset{\substack{|\\ SC_2H_5}}{C}\text{-}NH\text{-}N{=}C(CH_3)_2 \qquad (Xa\text{-}1)$$

bzw.

$$HN{=}\underset{\substack{|\\ SC_2H_5}}{C}\text{-}\overset{\substack{CO\text{-}NH\text{-}CO\text{-}O\text{-}C_6H_5\\ |}}{N}\text{-}N{=}C(CH_3)_2 \qquad (Xb\text{-}1)$$

Zu einer Lösung von 755 g (4,75 Mol) S-Ethyl-1-isopropyliden-isothiosemicarbazid in 7,5 Liter Toluol tropft man unter Eiskühlung 774 g (4,75 Mol) Phenoxycarbonylisocyanat. Nach beendeter Zugabe rührt man weiter 2 Stunden bei Raumtemperatur, saugt den ausgefallenen Niederschlag ab und wäscht mit Toluol nach. Nach dem Trocknen bei 110°C und Umkristallisieren aus Aceton erhält man 1282 g (83,8 % der Theorie) einer Verbindung, die nach DC, $^1$H-NMR und $^{13}$C-NMR einheitlich ist, wobei jedoch nicht entschieden werden kann, ob Struktur (Xa-1) oder (Xb-1) vorliegt. Die Verbindung schmilzt bei 159-160°C.

Alternativ erhält man diese Verbindung, wenn man eine Lösung von 514 g (2,0 Mol) N,N-Bisphenoxycarbonyl-amin in 2 Liter Toluol bei 90°C mit einer Lösung von 350 g (2,2 Mol) S-Ethyl-1-isopropyliden-isothiosemicarbazid in 750 ml Toluol versetzt, wobei die Temperatur der Mischung auf 67°C absinkt und nach wenigen Minuten das Reaktionsprodukt auszukristallisieren beginnt. Man rührt

weitere 30 Minuten bei 80°C, saugt den Niederschlag aus der erkalteten Reaktionsmischung bei 15°C ab, wäscht mit kaltem Toluol nach und trocknet bei 110°C. Gesamtausbeute nach Aufarbeiten der Mutterlauge: 707,5 g (78,8 % der Theorie).
Schmelzpunkt: 159-160°C.

Beispiel II-3:

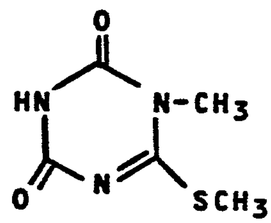

Zu einer Suspension von 715 g (3,08 Mol) N,S-Dimethylisothioharnstoff-hydroiodid und 720 g (2,8 Mol) N,N-Bisphenoxycarbonyl-amin in 2,8 Liter Isopropanol gibt man 311 g (3,08 Mol) Triethylamin und kocht 4 Stunden unter Rückfluß. Das ausgefallene Reaktionsprodukt wird bei 55°C abgesaugt, mit kaltem Isopropanol nachgewaschen und getrocknet. Man erhält 428 g (88 % der Theorie) an 1-Methyl-6-methylthio-1,2,3,4-tetrahydro-1,3,5-triazin-2,4-dion vom Schmelzpunkt 248°C bis 250°C.

Beispiel II-4

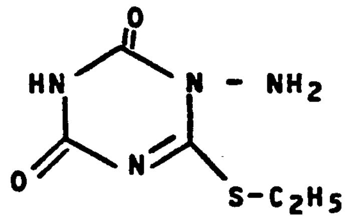

Eine Lösung von 114 g (0,5 Mol) 6-Ethylthio-3-isopropylidenimino-1,2,3,4-tetrahydro-1,3,5-triazin-2,4-dion in 2 Liter Isopropanol wird bei 80°C mit 4,8 g (0,025 Mol) p-Toluolsulfonsäurehydrat und 18 ml Wasser versetzt. Man rührt eine Stunde bei 80°C und engt dann bei 200 bis 300 mbar auf ein Drittel des Volumens ein. Das ausgefallene Reaktionsprodukt wird bei 0°C abgesaugt und mit kaltem Methanol nachgewaschen. Man erhält 92,5 g (98,4 % der Theorie) an 1-Amino-6-ethylthio-1,2,3,4-tetrahydro-1,3,5-triazin-2,4-dion vom Schmelzpunkt 209-210°C.

Beispiel II-5

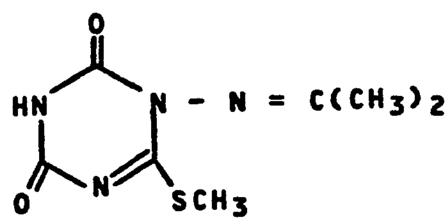

Eine Lösung von 72,5 g (0,5 Mol) S-Methyl-1-isopropyliden-isothiosemicarbazid in 750 ml Toluol wird unter Eiskühlung mit 81,5 g (0,5 Mol) Phenoxycarbonylisocyanat

0154906

versetzt, nach beendeter Zugabe eine Stunde unter Rückfluß gekocht und nach Erkalten abgesaugt. Nach Umkristallisieren des Rohproduktes aus Methanol erhält man 20,9 g (19,5 % der Theorie) an 1-Isopropylidenimino-6-methylthio-1,2,3,4-tetrahydro-1,3,5-triazin-2,4-dion vom Schmelzpunkt 190°C bis 191°C.

## Anwendungsbeispiele

In den folgenden Anwendungsbeispielen wurde die nachstehend aufgeführte Verbindung als Vergleichssubstanz herangezogen:

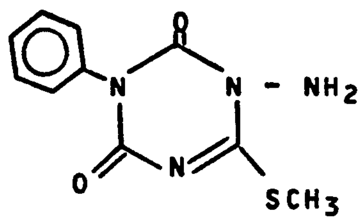

(A)

1-Amino-6-methylthio-3-phenyl-(1H, 3H)-1,3,5-triazin-2,4-dion

(bekannt aus DE-OS 22 54 200)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Kulturpflanzenselektivität gegenüber dem Stand der Technik zeigen in diesem Test z.B. die Verbindungen gemäß folgender Herstellungsbeispiele: 6, 13 und 16.

Patentansprüche

1. 3-Cyclopropylmethyl-1,3,5-triazin-2,4-dione der allgemeinen Formel (I),

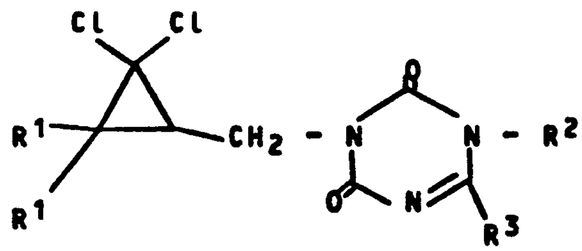

(I),

in welcher

$R^1$ für Wasserstoff oder Alkyl steht,

$R^2$ für Alkyl, für Amino oder für einen Rest

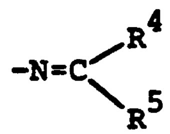 steht, wobei $R^4$ und $R^5$ unabhängig voneinander für Wasserstoff, für Alkyl oder für gegebenenfalls substituiertes Aryl stehen, oder gemeinsam für einen zweifach verknüpften Alkandiylrest stehen, und

$R^3$ für Alkylthio, Alkylamino oder Dialkylamino steht.

2. Verbindungen der allgemeinen Formel (I) gemäß Anspruch 1, dadurch gekennzeichnet, daß darin

0154906

$R^1$ für Wasserstoff oder für geradkettiges oder verzweigtes Alkyl mit bis zu 4 Kohlenstoffatomen steht,

$R^2$ für Amino, für geradkettiges oder verzweigtes Alkyl mit bis zu 4 Kohlenstoffatomen oder für einen Rest 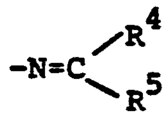 steht, wobei $R^4$ und $R^5$ jeweils unabhängig voneinander für Wasserstoff, für geradkettiges oder verzweigtes Alkyl mit bis zu 6 Kohlenstoffatomen oder für einfach oder mehrfach, gleich oder verschieden durch Halogen, Cyano, Nitro, sowie jeweils geradkettiges oder verzweigtes Alkyl, Alkoxy oder Alkylthio mit jeweils bis zu 4 Kohlenstoffatomen substituiertes Phenyl stehen oder gemeinsam für einen zweifach gebundenen Alkandiylrest mit 4 bis 6 Kohlenstoffatomen stehen und

$R^3$ für jeweils geradkettiges oder verzweigtes Alkylthio, Alkylamino oder Dialkylamino mit jeweils bis zu 6 Kohlenstoffatomen in den einzelnen Alkylteilen steht.

3. Verbindungen der allgemeinen Formel (I) gemäß Anspruch 1, dadurch gekennzeichnet, daß darin

$R^1$ für Wasserstoff, Methyl oder Ethyl steht,

0154906

$R^2$ für Methyl, Ethyl, Amino oder einen der Reste

$-N=C(CH_3)_2$, $-N=CH-C(CH_3)_3$, $-N=CH-C_6H_5$, -N=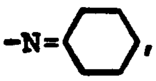,

-N=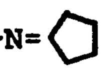 oder -N=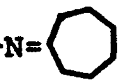 steht und

$R^3$ für Methylthio, Ethylthio, n- und i-Propylthio, n-, i-, s- und t-Butylthio, Methylamino, Ethylamino, Dimethylamino oder Diethylamino steht.

4. Verfahren zur Herstellung von 3-Cyclopropylmethyl-1,3,5-triazin-2,4-dionen der allgemeinen Formel (I) gemäß Anspruch 1, dadurch gekennzeichnet, daß man

(a) 1,3,5-Triazin-2,4-dione der Formel (II),

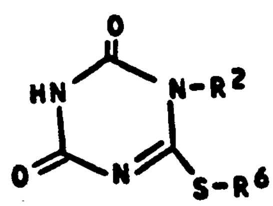

(II)

in welcher

$R^2$ die in Anspruch 1 angegebene Bedeutung hat und

$R^6$ für Alkyl steht,

mit Cyclopropylmethylhalogeniden der Formel (III),

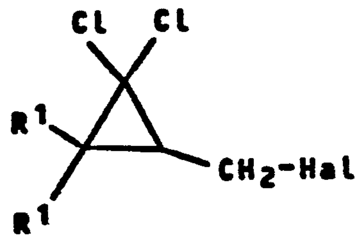 (III).

in welcher

$R^1$ die in Anspruch 1 angegebene Bedeutung hat und

Hal für Halogen steht,

gegebenenfalls in Gegenwart eines Verdünnungsmittels und gegebenenfalls in Gegenwart eines Säurebindemittels umsetzt,

oder daß man

(b) Isothioharnstoff-Derivate der Formel (IV),

$$H_2N - \underset{\underset{\displaystyle S - R^6}{|}}{C} = N - R^7 \qquad (IV)$$

in welcher

$R^6$ die oben angegebene Bedeutung hat und

$R^7$ für Alkyl oder für gegebenenfalls substituiertes Alkylidenimino bzw. Cycloalkylidenimino, insbesondere für Methyl, Ethyl oder einen der Reste $-N=C(CH_3)_2$, $-N=CH-C_6H_5$, $-N=CH-C(CH_3)_3$ oder -N= 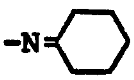 steht, oder

deren Hydrosalze mit Cyclopropylmethylamin-Derivaten der Formel (V),

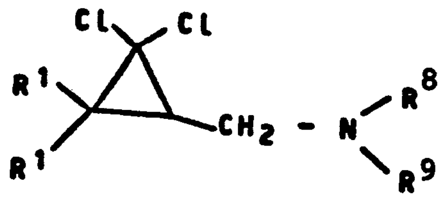

(V)

in welcher

$R^1$ die oben angegebene Bedeutung hat und

$R^8$ und $R^9$ unabhängig voneinander jeweils für gegebenenfalls substituiertes Aryloxycarbonyl stehen,

gegebenenfalls in Gegenwart eines Verdünnungsmittels und gegebenenfalls in Gegenwart eines Säurebindemittels umsetzt oder daß man

(c) die nach Verfahren (a) oder (b) erhältlichen 1-Alkylidenimino-3-cyclopropylmethyl-1,3,5-triazin-2,4-dione der Formel (Ia),

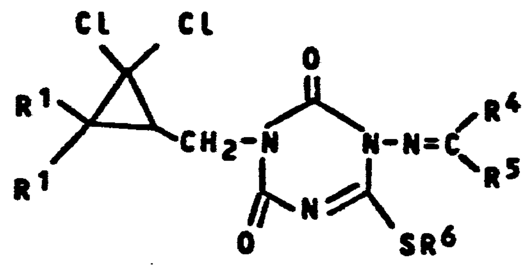

(Ia)

in welcher

$R^1$, $R^4$, $R^5$ und $R^6$ die oben angegebene Bedeutung haben,

entweder in prinzipiell bekannter Weise mit Säuren oder alternativ mit Isothiosemicarbaziden der Formel (IVa),

$$H_2N - \underset{\underset{SR^6}{|}}{C} = N - NH_2 \qquad \text{(IVa)}$$

in welcher

$R^6$ die oben angegebene Bedeutung hat,

oder deren Hydrosalzen gegebenenfalls in Gegenwart eines Verdünnungsmittels und gegebenenfalls in Gegenwart eines Katalysators umsetzt zu den 1-Amino-3-cyclopropylmethyl-1,3,5-triazin-2,4-dionen der Formel (Ib),

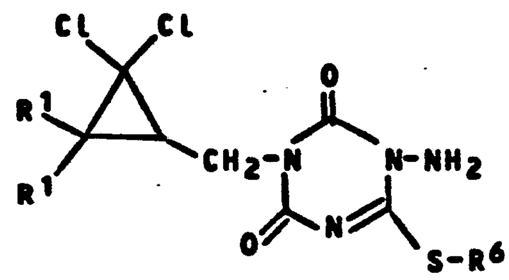

(Ib)

in welcher

$R^1$ und $R^6$ die oben angegebene Bedeutung haben,

oder daß man

(d) die nach Verfahren (a), (b) oder (c) erhältlichen 6-Alkylthio-3-cyclopropylmethyl-1,3,5-triazin-2,4-dione der Formel (Ic),

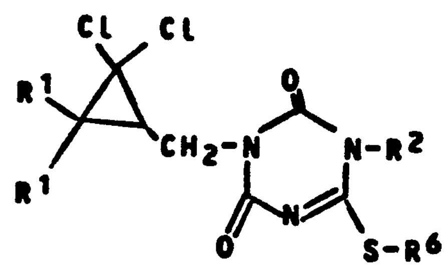

(Ic)

in welcher

$R^1$, $R^2$ und $R^6$ die oben angegebene Bedeutung haben,

mit Aminen der Formel (VI),

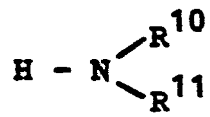

(VI)

in welcher

$R^{10}$ für Wasserstoff oder Alkyl steht und

$R^{11}$ für Alkyl steht,

gegebenenfalls in Gegenwart eines Verdünnungsmittels umsetzt zu den 6-Amino-3-cyclopropylmethyl-1,3,5-triazin-2,4-dione der Formel (Id),

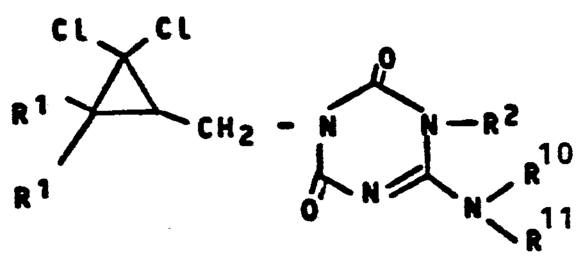

(Id)

in welcher

$R^1$, $R^2$, $R^{10}$ und $R^{11}$ die oben angegebene Bedeutung haben,

oder daß man

(e) die nach Verfahren (a), (b) oder (c) erhältlichen 1-Amino-3-cyclopropylmethyl-1,3,5-triazin-2,4-dione der Formel (Ie),

 

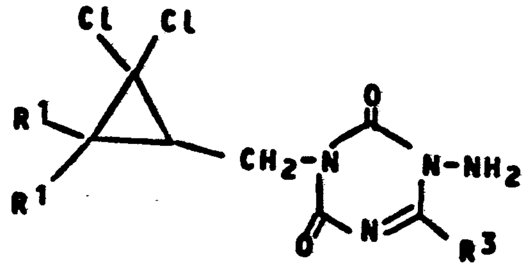

(Ie)

in welcher

$R^1$ und $R^3$ die oben angegebene Bedeutung haben,

mit Aldehyden oder Ketonen der Formel (VII),

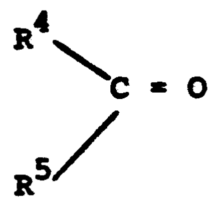

(VII)

in welcher

$R^4$ und $R^5$ die oben angegebene Bedeutung haben,

gegebenenfalls in Gegenwart eines Verdünnungsmittels und gegebenenfalls in Gegenwart eines Katalysators oder Reaktionshilfsmittels umsetzt zu den 1-Alkylidenimino-3-cyclopropylmethyl-1,3,5-triazin-2,4-dionen der Formel (If)

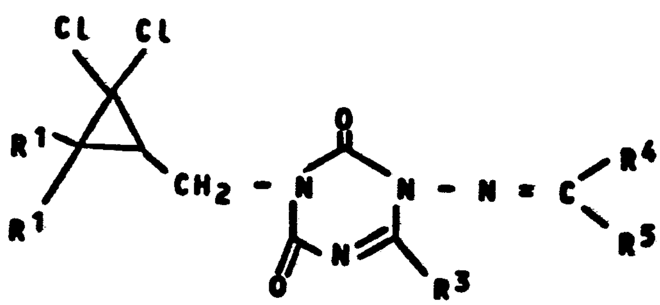

(If)

in welcher

$R^1$, $R^3$, $R^4$ und $R^5$ die oben angegebene Bedeutung haben.

5. Herbizide Mittel, gekennzeichnet durch einen Gehalt an mindestens einem 3-Cyclopropylmethyl-1,3,5-triazin-2,4-dion der Formel (I) gemäß Anspruch 1.

6. Verwendung von 3-Cyclopropylmethyl-1,3,5-triazin-2,4-dionen der Formel (I) gemäß Anspruch 1 zur Bekämpfung von unerwünschtem Pflanzenwachstum.

7. Verfahren zur Herstellung von herbiziden Mitteln, dadurch gekennzeichnet, daß man 3-Cyclopropylmethyl-1,3,5-triazin-2,4-dione der Formel (I) gemäß Anspruch 1 mit Streckmitteln und/oder oberflächenaktiven Mitteln vermischt.

8. 1,3,5-Triazin-2,4-dione der Formel (II),

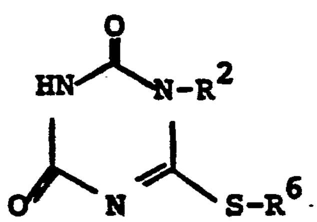

(II)

in welcher

$R^2$ für Alkyl, für Amino oder für einen Rest

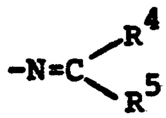 steht, wobei $R^4$ und $R^5$ unabhängig voneinander für Wasserstoff, für Alkyl oder für gegebenenfalls substituiertes Aryl stehen, oder gemeinsam für einen zweifach verknüpften Alkandiylrest stehen, und

$R^6$ für Alkyl steht.